# EUROPEAN PATENT APPLICATION

(11) **EP 4 434 553 A1**
(43) Date of publication of application: **25.09.2024**
(21) Application number: 23163652.3
(22) Date of filing: 23.03.2023
(51) Int. Cl.: A61M 1/06

(54) **BREAST PUMP SYSTEM**

(71) Applicant: Koninklijke Philips N.V., 5656 AG Eindhoven (NL)
(72) Inventor: VAN DER KOOI, Johannes Tseard, Eindhoven (NL); LIPSCH, Job, Eindhoven (NL); CLAASSEN, Coen Petrus Martinus, Eindhoven (NL)
(74) Representative: Philips Intellectual Property & Standards

(57) **Abstract**

A breast pump system comprises an expression kit, a pump, and a buffer. A valve selectively provides an interconnection between two of the pump, the buffer, and the expression kit. The valve is controlled in a first type of phase to couple the buffer to the expression kit, in a second type of phase to couple the expression kit to the activated pump and in a third type of phase to couple the expression kit to the pump and to vent the kit pressure. These different phases enable a cyclic pressure waveform to be generated for the two kits in which the energy consumption is reduced by using a buffer to reduce the venting of pressure during the cycle.

## Description

### FIELD OF THE INVENTION

This invention relates to breast pump systems, for example with an expression kit for each breast.

### BACKGROUND OF THE INVENTION

Breast pumps are used by breast feeding women to extract milk such that the extracted milk can be fed to their babies at a later time.

Typically, the breast is placed into a funnel shaped cup and a vacuum is applied such that milk is extracted. The breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

Wearable breast pumps are characterized in that they can be fully worn on the user's body, allowing the user to move around freely. Some of these are small enough to fit into a user's bra. A motor, battery and milk container are for example integrated to form a single unit.

Often, it is desirable to express both breasts at the same time. A user will then typically acquire a set of identical devices (e.g. in one housing) to achieve a setup which performs stimulation and expression independently for each breast.

This results in the use of two pumps, which is costly both in terms of components as well as power consumption, and it may also result in a high level of noise.

WO 2020/007671 discloses a double breast pump device which enables vacuum to be transferred between two expression kits. Thus, the volume of one expression kit is used as a storage volume of under pressure for the other expression kit. However, a complicated valve arrangement is needed.

WO 2021/244863 discloses a breast pump system in which an expression kit, a volume element (for storing a vacuum) and a pump are coupled together by a valve arrangement. The vacuum generated by the expression kit can again be reused instead of being vented to the atmosphere.

The known designs for re-using stored pressure have complicated and/or costly valve designs. It would be desirable to enable a simpler and/or lower cost configuration.

### SUMMARY OF THE INVENTION

The invention is defined by the claims.

According to examples in accordance with an aspect of the invention, there is provided a breast pump system comprising:
an expression kit;
a buffer;
a pump for delivering a negative pressure, wherein the pump has a fully activated mode for delivering the negative pressure and a non-fully-activated mode;
a valve which selectively provides an interconnection between a selected two of the pump, the buffer, and the expression kit; and
a controller for controlling the valve and the pump,
wherein the controller is adapted to control the valve:
   in a first type of phase to couple the buffer to the expression kit;
   in a second type of phase to couple the expression kit to the activated pump; and
   in a third type of phase to couple the expression kit to the non-fully-activated pump and to vent the kit pressure.

In the first type of phase, the pressure is shared between the buffer and the expression kit. In some uses of this phase, this decreases the kit pressure (i.e. increases the level of vacuum) when the buffer was previously at a lower pressure than the expression kit, and in other uses of this phase this increases the expression kit pressure (i.e. decreases the level of vacuum) when the buffer was previously at a higher pressure than the expression kit.

In the second type of phase, the expression kit can be pumped to a maximum vacuum level with the pump on.

In the third type of phase, the expression kit pressure may be vented, but using the same valve configuration as the second type of phase.

In this way, a simple single valve enables reuse of vacuum pressure, by using a buffer as a temporary pressure store.

The system for example comprises a second expression kit, wherein the expression kit and the second expression kit are supplied with the same pressure. Thus, the system may be used with a single expression kit or with a pair of expression kits. When two expression kits are used, they are coupled together so that their pressure cycles are synchronized.

The valve for example comprises a (single) three-way valve.

The venting of the kit pressure may be achieved by using leakage through the pump or by using a separate pneumatic valve, as will be clear from the examples below.

The non-fully-activated mode is a mode in which the pump is not being used to deliver negative pressure directly to the expression kit. It may be fully turned off, but depending on the implementation, it may not need to be fully turned off.

The controller is for example adapted to control the valve and the pump in a four-phase cycle, wherein the four phases comprise:
a first phase of the first type;
a second phase of the second type;
a third phase of the first type; and
a fourth phase of the third type

The four phase cycle thus involves (i) sharing the buffer pressure (which is at an intermediate pressure level from the end of the fourth phase) with the expression kit (which was previously vented) to lower the expression kit pressure (i.e. increase the vacuum level), (ii) using the pump to lower the expression kit pressure further to the maximum vacuum pressure, (iii) sharing the buffer pressure with the expression kit to raise the pressure back to an intermediate level, and (iv) venting the expression kit while retaining the buffer at the intermediate pressure level.

The controller is preferably adapted to deactivate the pump in the first and third phases. In these phases, the pump is isolated from the expression kit and buffer by the valve.

In one example, the pump provides a pressure leakage path for said venting of the kit pressure to atmospheric conditions in the non-fully activated mode. In this way, the pump itself provides the venting of the expression kit pressure, in the third type of cycle. The pump may thus be deactivated in the fourth phase. This avoids the need for additional components to provide a venting function.

In another example, the pump functions as a stop in the non-fully-activated mode, wherein the breast pump system further comprises a pneumatic valve which provides a pressure leakage path in parallel with the pump for said venting of the kit pressure, the pneumatic valve being opened by the pressure of the buffer. By "stop" is meant a closed connection so that the pressure is not allowed to leak substantially (i.e. small leaks can be tolerated).

In this way, an additional valve enables the venting of the kit pressure, in the third type of phase. This avoids the need for a pump with a designed leakage path.

The pneumatic valve may have a user-adjustable trigger pressure. This enables the pneumatic valve to be adjusted to different user-settable vacuum levels to be applied to the expression kit or kits.

The controller is in this example preferably adapted to control the valve in a four phase cycle, wherein the four phases comprise:
a first phase of the first type, with the pneumatic valve closing;
a second phase of the second type with the pneumatic valve closed;
a third phase of the first type with the pneumatic valve opening; and
a fourth phase of the third type with the pneumatic valve open.

The four phase cycle thus involves sharing the buffer pressure (which is at an intermediate pressure level from the end of the fourth phase) with the kits (which were previously vented) to lower the kit pressure, using the pump to lower the kit pressure further to the maximum vacuum pressure, sharing the buffer pressure with the kits to raise the pressure back to an intermediate level, and venting the kits using the pneumatic valve while retaining the buffer at the intermediate pressure level.

The invention also provides a method of controlling a breast pump system for the non-therapeutic expression of milk, wherein the breast pump system has an expression kit, a buffer, a pump for delivering a negative pressure wherein the pump has a fully activated mode for delivering the negative pressure and a non-fully-activated mode, a valve which selectively provides an interconnection between a selected two of the pump, the buffer, and the expression kit, and a controller for controlling the valve and the pump,
wherein the method comprises:
operating a controller to control the pump and the valve in:
   a first type of phase to couple the buffer to the expression kit;
   a second type of phase to couple the expression kit to the fully activated pump; and
   a third type of phase to couple the expression kit to the non-fully activated pump and to vent the kit pressure.

The method for example comprises using the controller to control the valve and the pump in a four phase cycle, wherein the four phases comprise:
a first phase of the first type;
a second phase of the second type;
a third phase of the first type; and
a fourth phase of the third type

The invention also provides a computer program comprising computer program code means which is adapted, when said program is run on a controller of a breast pump system, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a known breast pump system;
Fig. 2 shows a mother wearing two wearable breast pumps;
Fig. 3 shows a breast pump system having a pump shared between two expression kits;
Fig. 4 shows a first example of breast pump system;
Fig. 5 shows a pressure waveform for the operation of the breast pump system of Fig. 4;
Fig. 6 shows a second example of breast pump system;
Fig. 7 shows a pressure waveform for the operation of the breast pump system of Fig. 6; and
Fig. 8 shows a valve design for use in the system of Fig. 6.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump system which comprises an expression kit, a pump, and a buffer. A valve selectively provides an interconnection between two of the pump, the buffer, and the expression kit. The valve is controlled in a first type of phase to couple the buffer to the expression kit, in a second type of phase to couple the expression kit to the activated pump and in a third type of phase to couple the expression kit to the pump and to vent the kit pressure. These different phases enable a cyclic pressure waveform to be generated for the two kits in which the energy consumption is reduced by using a buffer to reduce the venting of pressure i.e. air during the cycle.

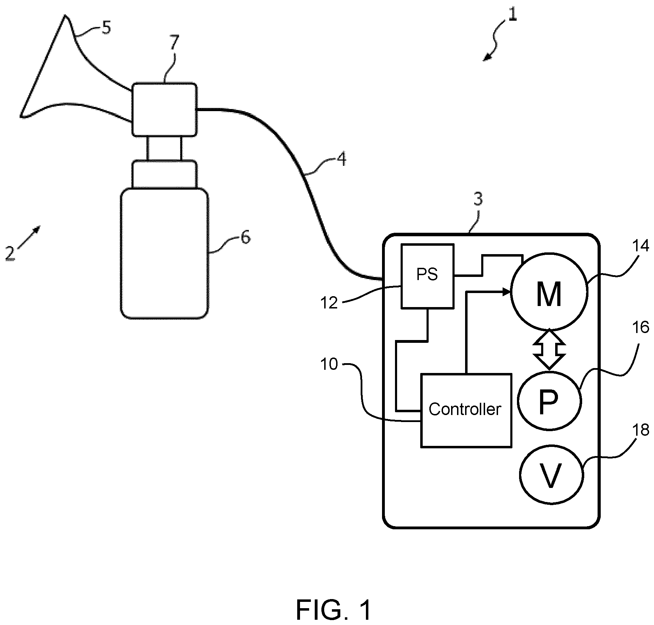

Fig. 1 shows a known breast pump system 1, comprising a single expression kit 2 and a pump arrangement 3 which are connected via a tube 4. The pump arrangement includes various components in addition to the pump, so may be considered to be a general operating unit.

The expression kit 2 is formed with a main body 7, a funnel 5 for receiving a breast of a user and a receptacle 6 for collecting the expressed milk. The funnel 5 and the receptacle 6 are connected to the main body 7. The main body 7 comprises a vacuum chamber. A flexible membrane or diaphragm is located in the vacuum chamber. The membrane prevents expressed milk from flowing into the tube 4 leading to the pump arrangement unit 3.

The pump arrangement 3 may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16. The vacuum pump for example comprises membrane/displacement pump driven by the motor. The controller controls 10 the operation of the power source 12 and motor 14 (and hence the vacuum pump 16). The pump arrangement 3 further comprises a vacuum venting component, such as a solenoid valve 18.

In use, the vacuum pump applies a vacuum to the membrane located in the main body 7 so that it deforms. The membrane deforms to create a vacuum in the funnel 5 which in turns applies a vacuum to the breast which enables milk to be expressed. Although the breast pump system 1 is described as comprising a membrane such that the vacuum is applied indirectly to the breast, it should be understood that in an alternative embodiment, the vacuum is applied directly to the breast of a user. In this case, the breast pump system does not comprise a membrane and the vacuum created by the vacuum pump is applied directly to the breast. The pump in this case will be cleanable or disposable.

The vacuum is applied to the breast at intervals. That is, a pressure differential is applied on a cyclic basis. In this known system, after a vacuum has been established, the pressure from the vacuum is vented by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression kit which enables milk to be expressed from the breast of a user. When the solenoid valve is opened, the vacuum generated by the vacuum pump is vented as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. In one type of design, a top part contains the drivetrain (pump, motor, controller) and a bottom part forms the collection vessel. This enables breast pumping to be performed more discretely. Of course, other designs are possible.

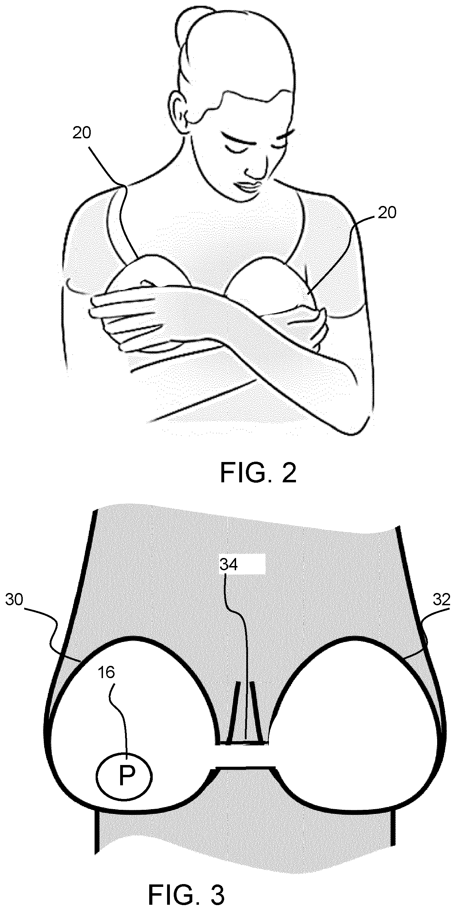

Fig. 2 shows a mother wearing two wearable breast pumps 20.

In such a design, the funnel 5 of Fig. 1 is implemented as breast shield, and this breast shield is received within an outer enclosure of the wearable breast pump.

The invention will be described with reference to a breast pump system having two expression kits. Each expression kit is an assembly for fitting over the breast of a user, and for example includes a breast shield and optionally also a membrane (known as the diaphragm) between the breast shield and the breast, so that the membrane limits the parts of the breast pump system that can be contaminated with milk. The invention may however also be applied to a single expression kit.

The invention relates in particular to reducing energy loss when venting an expression kit to the ambient surroundings.

Examples of the invention as applied to a breast pump system with two expression kits will be described.

Fig. 3 shows a breast pump system comprising a first expression kit 30 and a second expression kit 32. Each expression kit is a wearable device, and each expression kit includes a breast shield. A single pump is provided for operating the two expression kits. In the example shown, the pump is located at one expression kit and a coupling 34 is provided between the expression kits so that the single pump may deliver an under pressure to both expression kits. The expression kits may each comprise a milk sensor for sensing milk flow and for measuring flow volume.

In a typical arrangement, the expression kits are independent and identical. However, as shown in Fig. 3 it is also possible for expression kits to have different sets of components to reduce the duplication of hardware. For example, US 2020/155738 discloses a wearable breast pump with two expression kits, but only one has the controller or battery or pump to be used by both expression kits.

The preferred implementation of the invention relates to a design with a shared pump which is used by two expression kits as represented in Fig. 3. The overall system may be wearable (so that the pump is located at one of the expression kits) or it may be portable, for example with the pump 16 remote from the expression kits, for example mounted on a belt. When one expression kit has the pump, other components may be at the other kit, such as a controller or battery or valves, to balance the weight and size/shape of the two expression kits.

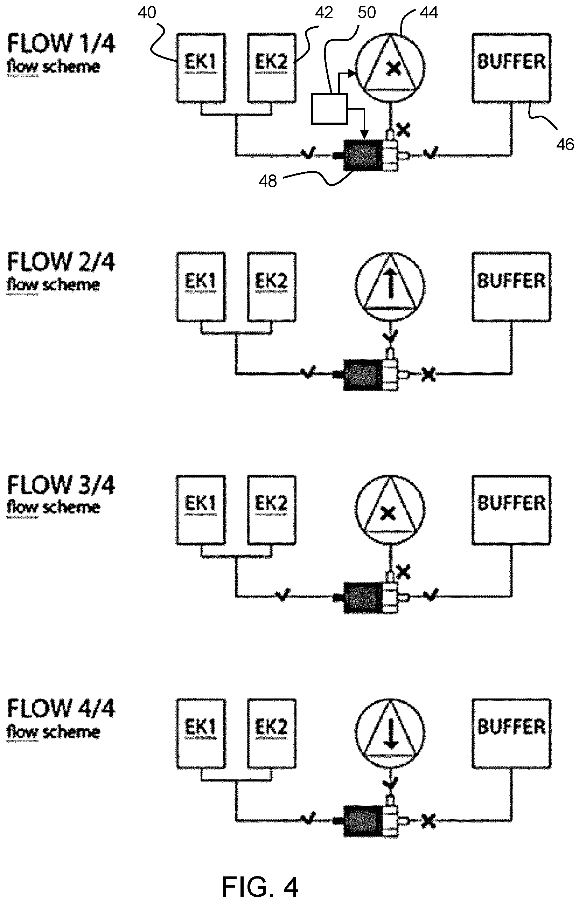

Fig. 4 shows a first example of a breast pump system comprising a first expression kit 40, a second expression kit 42 and a buffer. The first and second expression kits connect to a shared pressure input so that the pressure cycle applied to the first and second expression kits is synchronized.

In this example, a shared pump 44 is used for delivering a negative pressure for both expression kits. The pump has a fully activate mode in which it delivers a maximum negative pressure. It has a non-fully-activated mode. This may be a mode with the pump fully turned off, or it may be a mode with the pump delivering a lower under-pressure.

A valve 48 selectively provides an interconnection between a selected two of the pump 44, the buffer 46, and the pair of expression kits 40, 42. A controller 50 is provided for controlling the valve and the pump.

The system aims to deliver a cyclic pressure waveform to the expression kits. In particular, a cycle with four phases is provided. The valve and pump are controlled differently in the different phases. In particular, there is a first type of phase to couple the buffer to the expression kits (and isolate the pump), a second type of phase to couple the expression kit to the activated pump (and isolate the buffer) and a third type of phase to couple the expression kits to the pump but with the pump now not fully activated, This third type of phase isolates the buffer. In this third type of phase, the expression kit is vented, to atmospheric pressure or to a baseline pressure. This venting may be achieved in different ways, and the example of Fig. 4 represents a first way to achieve the venting.

In the arrangement of Fig. 4, when the pump is in its non-fully activate mode (which in this case is a deactivated mode) it presents a pressure leakage path, by which the pump output is coupled to atmospheric pressure (or again to a selected baseline pressure for example by means of a passive control valve). In this way, the pump itself provides the venting of the expression kit pressure, in the third type of cycle. This avoids the need for additional components to provide a venting function.

Fig. 4 shows the four phases of the pressure cycle.

The first phase is of the first type. During this first phase, the pressure is shared between the buffer and the expression kits. The pump is in the non-fully-activated mode, for example deactivated. It could still however be running at a lower setting, since its output is in any case isolated by the three-way valve. In this first phase, this pressure sharing decreases the expression kit pressure (i.e. increases the level of vacuum) because the buffer was previously at a lower pressure than the expression kits once the system has reached an equilibrium cyclic operation (as will be clear from the description that follows).

The second phase is of the second type. During this second phase, the expression kits are pumped to a maximum vacuum level with the pump on. The pump is activated.

The pump may be controlled to deliver a single constant pressure, i.e., the maximum under pressure level. Thus, the creation of the cyclic pressure waveform is the result of the control of the valve and the use of the stored pressure in the buffer, rather than by control of the pump output waveform.

However, a waveform may also be applied to the output pressure generated by the pump to make the transition between the different phases more smooth. There also could be a small overlap between the different phases to make transition more smooth.

The third phase is of the first type, so that the pressure is again shared between the buffer and the expression kits. However, in this case, this increases kit pressure (i.e. decreases the level of vacuum) because the expression kits had been driven by the pump to a lower pressure (deeper vacuum) than the buffer in the previous phase of the cycle. The pump is deactivated.

The fourth phase is of the third type. The expression kit pressure is vented, in this example by the leakage through the pump in its deactivated state. The valve is in the same configuration as the second type of phase.

The four phase cycle thus involves (i) sharing the buffer pressure (which is at an intermediate pressure level from the end of the fourth phase) with the expression kit or kits (which were previously vented) to lower the kit pressure (i.e. increase the vacuum level), (ii) using the pump to lower the kit pressure further to the maximum vacuum pressure, (iii) sharing the buffer pressure with the kits to raise the pressure back to an intermediate level, and (iv) venting the kits while retaining the buffer at the intermediate pressure level. This intermediate pressure level is then the start of the first phase, discussed above.

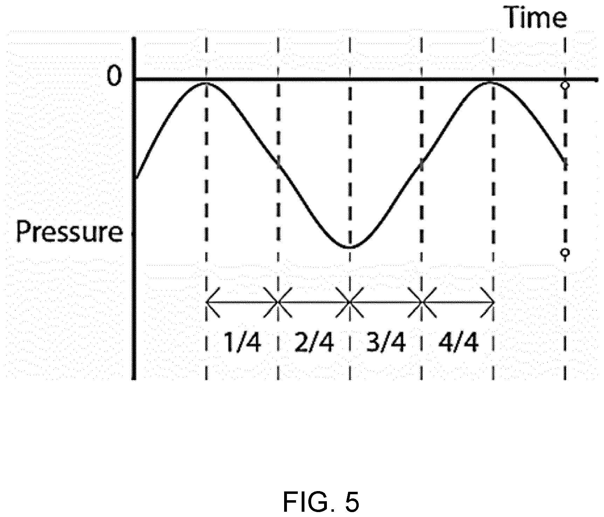

Fig. 5 shows the resulting pressure waveform applied to the expression kits using the approach described above.

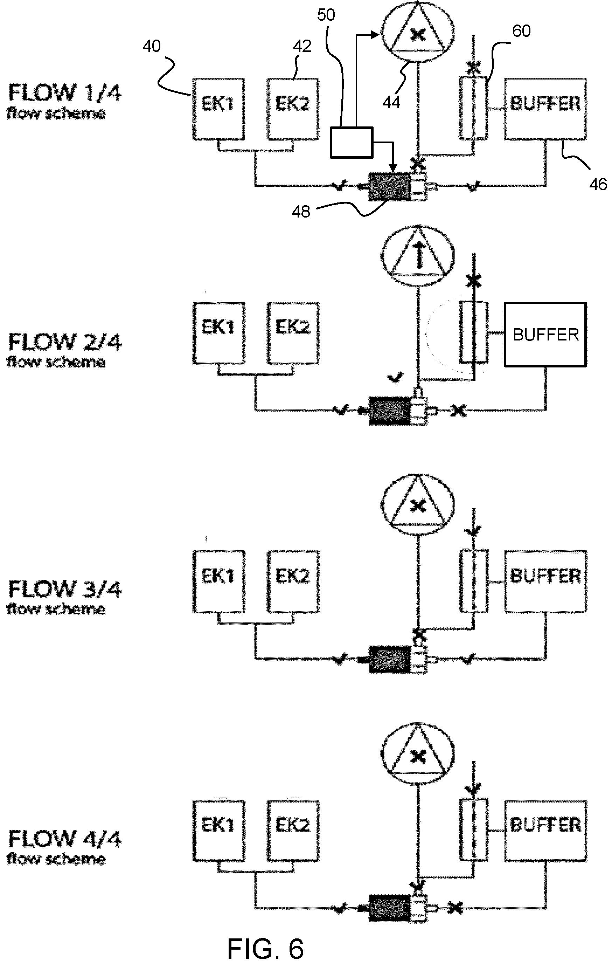

Fig. 6 shows a second example of a breast pump system, again comprising a first expression kit 40, a second expression kit 42 and a buffer 46. The first and second expression kits connect to a shared pressure input so that the pressure cycle applied to the first and second expression kits is synchronized. In this example, a single pump 44 is again used for delivering a negative pressure. A valve 48 selectively provides an interconnection between a selected two of the pump 44, the buffer 46, and the pair of expression kits 40, 42. A controller 50 is provided for controlling the valve and the pump.

The difference of this example to Fig. 4 is that the breast pump system further comprises a pneumatic valve 60 which provides a pressure leakage path in parallel with the pump 44. The pneumatic valve is opened by the pressure of the buffer. The pump then does not need to provide a leakage path. Instead, the pump has an activated mode for delivering the negative pressure and a deactivated mode when it functions as a stop (although some leakage is permitted).

The controller is again preferably adapted to control the valve in a four phase cycle, wherein the four phases as shown in Fig. 6.

The first phase is of the first type. During this first phase, the pressure is shared between the buffer and the expression kits. The pump is in the non-fully-activated mode, for example deactivated. It could still however be running at a lower setting, since its output is in any case isolated by the three-way valve. In this first phase, this pressure sharing decreases the expression kit pressure (i.e., increases the level of vacuum) because the buffer was previously at a lower pressure than the expression kit (as will be clear from the description that follows). The resulting increasing pressure of the buffer serves to close the pneumatic valve, so it is closing during the first phase.

The pneumatic valve is open when the pressure is sufficiently low, i.e. the vacuum is sufficiently strong, in this example.

The second phase is of the second type. During this second phase, the expression kits are pumped to a maximum vacuum level with the pump on. The pump is thus activated. The pneumatic valve remains closed because the buffer pressure has not changed.

The third phase is of the first type, so that the pressure is again shared between the buffer and the expression kits. However, in this case, this increases the expression kit pressure (i.e. decreases the level of vacuum) because the expression kits had been driven by the pump to a lower pressure (deeper vacuum) than the buffer in the previous phase of the cycle. The pump is in the non-fully-activated mode, for example deactivated. It could again be running at a lower setting, since its output is in any case isolated by the three-way valve.

The pressure sharing decreases the buffer pressure, (i.e. creates a stronger vacuum) so that as a result the pneumatic valve opens, but the three-way valve isolates the pneumatic valve from the expression kits. The resulting decreasing pressure of the buffer thus serves to open the pneumatic valve, so it is opening during the third phase.

The fourth phase is of the third type. The expression kit pressure is vented by the pneumatic valve. The pneumatic valve remains open but this time the three-way valve provides a connection to the expression kits.

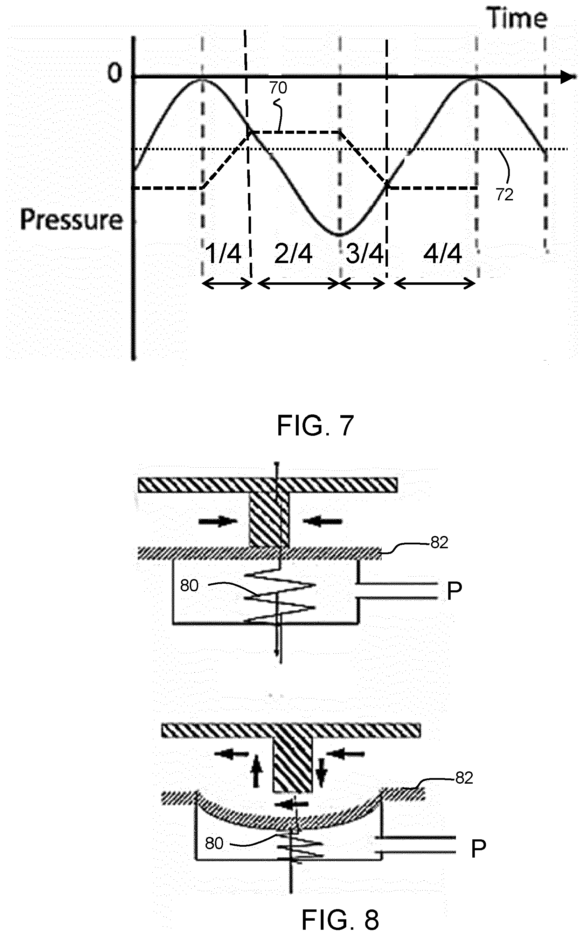

Fig. 7 shows the resulting pressure waveform applied to the expression kits using the approach described above. The dotted waveform 70 shows the buffer pressure and the line 72 show the pressure threshold below which the pneumatic valve opens (i.e. the pneumatic valve opens when there is sufficient vacuum in the buffer). A higher pressure (lower vacuum) for the threshold corresponds to a better start up phase.

It can be seen that the valve is open in phase 4/4, transitions from open to closed in phase 1/4 and transitions from closed to open in phase 3/4.

Fig. 8 shows an example of a possible pneumatic valve in closed configuration (top image) and open configuration (bottom image). The valve comprises a spring 80 which closes a diaphragm 82. There is a force on the diaphragm from the spring and also from the internal pressure P of a chamber which is closed by the diaphragm. In this example, a sufficiently large negative pressure P will open the valve against the bias of the spring.

A correct spring force is needed to open and close the valve in response to the pressure fluctuation in the buffer. This pressure fluctuation mainly depends on a pressure setting of the expression kits. When the user sets a different pressure setting for pumping, the spring force needs to be adjusted. This can be done for example by an adjusting mechanism that reduces or increases the spring's pretension. In this way, the pneumatic valve may have a user-adjustable trigger pressure so that the pneumatic valve can be adjusted to different user-settable vacuum levels to be applied to the expression kits.

The pump used in the examples above is for example a radial fan connected to an electric motor. Other solutions may however be more energy efficient and lower in noise, such as using the rotation of a motor in a dynamic or static manner to open or close a valve system that is integrated into the more generic pumps used in breast pumps. Those pumps involve for example displacement pumps like membrane pumps or piston pumps. In case of piston pumps, a valve system driven by camshaft may be used.

For the first example of Fig. 4, the timing of the operation of the three-way valve and the timing of the operation of the pump may be configured to achieve a desired pressure waveform. For example, the pump may be turned on and off at different times to the turning on and off of the valve. Thus, the different phases may be considered to overlap in that the switching functions between the phases do not need to take place at exactly the same time.

Similarly, for the second example of Fig. 6, the timing of the operation of the three-way valve and the timing of the operation of the pneumatic valve may be configured to achieve a desired pressure waveform. The timing of the operation of the pneumatic valve is for example adapted by changing the pressure threshold 72 at which it switches. This may be achieved by changing the spring force of a spring valve. Again, the pneumatic valve may be turned on and off at different times to the turning on and off of the three-way valve.

These measures for example enable a smoother kit pressure waveform to be generated.

The pressure waveform will depend on the relative volumes of the expression kit or kits and the buffer. Typically, they have approximately the same volume. However, the relative volumes may also be designed to achieve a desired kit pressure waveform. The buffer volume could also be adaptable by the user. The user may for example be able to use the buffer for one or two expression kits, and adapt the volume accordingly. The buffer volume may also be used to control the level of energy gain or the characteristics of the resulting pressure profile.

The waveforms above are for a steady state cyclic operation of the system. In practice, there will be an initial settling time, since the first phase will initially start with ambient pressure in the buffer. Thus, the system will take some cycles for the buffer to reach its steady state pressure levels (e.g. in phases 2/4 and 4/4 of Fig. 7).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump system comprising:
an expression kit (40);
a buffer (46);
a pump (44) for delivering a negative pressure, wherein the pump has a fully activated mode for delivering the negative pressure and a non-fully-activated mode;
a valve (48) which selectively provides an interconnection between a selected two of the pump, the buffer, and the expression kit; and
a controller (50) for controlling the valve and the pump,
wherein the controller is adapted to control the valve:
in a first type of phase to couple the buffer to the expression kit;
in a second type of phase to couple the expression kit to the activated pump; and
in a third type of phase to couple the expression kit to the non-fully-activated pump and to vent the kit pressure.

2. The system of claim 1, comprising a second expression kit (42), wherein the expression kit and the second expression kit are supplied with the same pressure.

3. The system of claim 1 or 2, wherein the valve (48) comprises a three-way valve.

4. The system of any one of claims 1 to 3, wherein the controller is adapted to control the valve and the pump in a four phase cycle, wherein the four phases comprise:
a first phase of the first type;
a second phase of the second type;
a third phase of the first type; and
a fourth phase of the third type

5. The system of claim 4, wherein the controller is adapted to deactivate the pump in the first and third phases.

6. The system of any one of claims 1 to 5, wherein the pump provides a pressure leakage path for said venting of the kit pressure in the non-fully activated mode.

7. The system of any one of claims 1 to 5, wherein the pump functions as a stop in the non-fully-activated mode, wherein the breast pump system further comprises a pneumatic valve (60) which provides a pressure leakage path in parallel with the pump for said venting of the kit pressure, the pneumatic valve being opened by the pressure of the buffer.

8. The system of claim 7, wherein the pneumatic valve (60) has a user-adjustable trigger pressure.

9. The system of claim 7 or 8, wherein the controller is adapted to control the valve in a four-phase cycle, the four phases comprises:
a first phase of the first type, with the pneumatic valve closing;
a second phase of the second type with the pneumatic valve closed;
a third phase of the first type with the pneumatic valve opening; and
a fourth phase of the third type with the pneumatic valve open.

10. A method of controlling a breast pump system for the non-therapeutic expression of milk, wherein the breast pump system has an expression kit, a buffer, a pump for delivering a negative pressure wherein the pump has a fully activated mode for delivering the negative pressure and a non-fully-activated mode, a valve which selectively provides an interconnection between a selected two of the pump, the buffer, and the expression kit, and a controller for controlling the valve and the pump,
wherein the method comprises:
operating a controller to control the pump and the valve in:
a first type of phase to couple the buffer to the expression kit;
a second type of phase to couple the expression kit to the fully activated pump; and
a third type of phase to couple the expression kit to the non-fully activated pump and to vent the kit pressure.

11. The method of claim 10, comprising using the controller to control the valve and the pump in a four-phase cycle, wherein the four phases comprise:
a first phase of the first type;
a second phase of the second type;
a third phase of the first type; and
a fourth phase of the third type

12. A computer program comprising computer program code means which is adapted, when said program is run on a controller of a breast pump system, to implement the method of claim 10 or 11.
